# EUROPÄISCHE PATENTSCHRIFT

(11) **EP 2 268 610 B1**
(45) Veröffentlichungstag und Bekanntmachung des Hinweises auf die Patenterteilung: **03.08.2016**
(21) Anmeldenummer: 09727771.9
(22) Anmeldetag: 27.03.2009
(51) Int. Cl.: C07C 273/18, C07C 275/06, C07D 251/18, C07D 251/70

(54) **VERFAHREN ZUR HERSTELLUNG EINER VERBINDUNG MIT MINDESTENS EINER ZUMINDEST EINFACH SUBSTITUIERTEN AMINOGRUPPE**
METHOD FOR PRODUCING A COMPOUND WITH AT LEAST ONE AT LEAST MONOSUBSTITUTED AMINO GROUP
PROCÉDÉ DE PRÉPARATION D'UN COMPOSÉ AYANT AU MOINS UN GROUPE AMINO AU MOINS MONOSUBSTITUÉ

(30) Priorität: 31.03.2008 DE 102008016964
(43) Veröffentlichungstag der Anmeldung: 05.01.2011
(73) Patentinhaber: Borealis Agrolinz Melamine GmbH, 4021 Linz (AT)
(72) Erfinder: DICKE, Rene, A-4060 Leonding (AT); ENDESFELDER, Andreas, A-4020 Linz (AT); BURGER, Martin, 84032 Altdorf (DE); HAHN, Christoph, 4040 Linz (AT); SCHWARZINGER, Clemens, A-4060 Weis (AT); FÜRST, Wolfgang, A-4060 Leonding (AT); SCHMIDT, Harald, A-4040 Linz (AT)
(74) Vertreter: Maikowski & Ninnemann Patentanwälte Partnerschaft mbB
(86) Internationale Anmeldenummer: PCT/EP2009/002430
(87) Internationale Veröffentlichungsnummer: WO 2009/121603

(56) Entgegenhaltungen:
- EP-A- 0 125 616
- EP-A- 0 711 760
- EP-A- 0 882 720
- EP-A- 1 057 821
- WO-A-03/106558
- ROELL H ET AL: "DECORATIVE AMINOPLAST LAMINATES", ABSTRACT BULLETIN OF THE INSTITUTE OF PAPER CHEMISTRY, THE INSTITUTE OF PAPER CHEMISTRY-LIBRARY. APPLETON, US, vol. 59, no. 3, 1 September 1988 (1988-09-01), page 328, XP000014310,

## Beschreibung

Die Erfindung betrifft ein Verfahren zur Herstellung einer Verbindung mit mindestens einer zumindest einfach substituierten Aminogruppe gemäß dem Oberbegriff des Anspruchs 1 und die Verwendung einer mit einem entsprechenden Verfahren hergestellten Verbindung gemäß den Ansprüchen 25 oder 27.

Die JP 2000-063369 A beschreibt ein Verfahren zur Alkylierung von Melamin, in dem Melamin mit einem Alkohol bei hohen Temperaturen in Gegenwart eines Metallkatalysators auf einem mikroporösen Träger umgesetzt wird. Dabei werden vor allem niedrig alkylierte Produkte hergestellt. Bei diesem Verfahren wird einerseits eine niedrige Selektivität der alkylierten Produkte und andererseits die Bildung von Cyanursäureestern als Nebenprodukten durch Hydrolyse bzw. Substitution der Aminogruppen beobachtet. Die Reaktion erfolgt unter einer Stickstoff-, Argon-, Wasserstoff- oder Kohlenmonoxidatmosphäre.

Die EP 0 711 760 A1 beschreibt die Alkylierung von Melamin durch Umsetzung von Melamin in Gegenwart eines Katalysators und einer Atmosphäre aus Argon, Stickstoff, Kohlenmonoxid oder einer Mischung aus Wasserstoff und Kohlenmonoxid. Es werden kein vollständiger Umsatz der Edukte und keine Selektivität bezüglich einzelner Produkte erreicht.

Die EP 1 057 821 A1 und EP 882 720 A1 beschreiben die Alkylierung von Melamin mit Alkoholen in Gegenwart von Katalysatoren und einer Stickstoff- oder Wassersoffatmosphäre. Es werden kein vollständiger Umsatz der Edukte und keine Selektivität bezüglich einzelner Produkte erreicht.

Shinoda et al. (Appl. Catalysis A: General 194-195 (2000), 375-381) beschreiben eine Methylierung von Melamin ausgehend von Methanol. Für die Katalyse wird ein Metall mit einem sauren Träger benutzt und die Reaktion wird unter Schutzgas- (Argon-) oder Wasserstoffatmosphäre durchgeführt. Die Reaktion erreicht zwar einen vollständigen Umsatz, jedoch nur nach sehr langen Reaktionszeiten. Das gebildete Produktspektrum enthält verschieden substituierte Methylmelamine.

Aus der WO 03/106558 A1 sind Aminoplastformmassen und Aminoplasterzeugnisse, die in einem Mehrstufenverfahren hergestellt werden, bei dem in einer ersten Verfahrenstufe Vorkondensate aus C1-C8-Aldehyden und Melaminderivaten mit C1-C8-Alkoholen verethert werden. Die Aminoplaste werden u.a. zur Herstellung von Laminaten verwendet. Hierfür werden die Polytriazinether zur Bepulverung von Cellulosevliesen verwendet und anschließend aufgeschmolzen. Die so hergestellten Prepregs werden übereinandergelegt und mit einem unbehandelten Cellulosevlies als Oberseite zu einem Formteil verpresst.

Es ist auch bekannt, dass Ammonia-Harnstoff-Melamin-Formaldehyd Harze zur Herstellung von Aminoplast-Laminaten verwendet werden können. Derartige Aminoplast-Laminate sind wiederum für die Möbelindustrie und als Wandverkleidungen einsetzbar (Roell et al., Decorative Aminoplast Laminates, Abstract Bulletin of the Institute of paper chemistry, Appleton, USA, Bd. 59, Nr. 3, 1. September 1988, Seite 328).

Der Erfindung liegt die Aufgabe zugrunde, Verbindungen mit zumindest einer einfach substituierten Aminogruppe bereitzustellen, die - bei Einführung mehrerer Substituenten in die jeweilige Verbindung - in hoher Selektivität bezüglich der Verteilung der Substituenten herstellbar sind.

Diese Aufgabe wird mit einem Verfahren mit den Merkmalen des Anspruchs 1 gelöst. Demnach wird eine Ausgangssubstanz, die mindestens eine Aminogruppe trägt, mit einem Alkohol umgesetzt, wobei der Reaktionsansatz flüssigen bzw. gelösten und/oder gasförmigen Ammoniak aufweist. Insbesondere weist die Gasphase des Reaktionsansatzes gasförmigen Ammoniak auf. Die Ausgangssubstanz und der Alkohol sind dabei Teil einer Reaktionsmischung. Auch Ammoniak kann, beispielsweise in gelöster Form, Teil der Reaktionsmischung sein. Die Reaktionsmischung kann beispielsweise eine Flüssigkeit oder eine Dispersion sein. Die Aminogruppe der Ausgangssubstanz weist zumindest ein direkt an den Stickstoff gebundenes Wasserstoffatom auf. Das heißt, diese Aminogruppe kann unsubstituiert oder einfach substituiert sein. Nach der Umsetzung mit dem Alkohol R-OH ist der Rest R statt des ursprünglichen Wasserstoffatoms an das Stickstoffatom der Aminogruppe gebunden. Mit anderen Worten ist das erfindungsgemäße Verfahren ein Verfahren zur Derivatisierung einer Aminogruppe.

Überraschenderweise hat sich gezeigt, dass der Einsatz von Ammoniak als Gas oder als Lösung in Alkoholen zu einer nahezu vollständigen Unterdrückung von Nebenprodukten führt und dass gleichzeitig die Selektivität der Alkylierungsreaktion signifikant ansteigt.

Insbesondere kann die Nebenproduktbildung durch Hydrolyse signifikant reduziert oder unterdrückt werden.

Bei aus dem Stand der Technik bekannten Alkylierungsreaktionen führt demgegenüber insbesondere die Anwesenheit von Luft und von Spuren von Wasser zur Hydrolyse der gebildeten Produkte, wobei die entsprechenden Alkoxycyanurate gebildet werden. Solche Alkoxycyanurate können auch gebildet werden, wenn Methanol gegen eine komplette Aminogruppe unter Freisetzung von Ammoniak substituiert wird. Dies wird beim erfindungsgemäßen Verfahren nicht beobachtet.

Um eine möglichst niedrige Nebenproduktbildung und Hydrolyse zu erreichen, beträgt das Stoffmengenverhältnis von Ammoniak zu Alkohol 0,1 bis 2, insbesondere 0,5 bis 1,5 und ganz besonders 0,8 bis 1,3. Die in der vorliegenden Anmeldung angegebenen Zahlenwerte sind sowohl bei einseitig offenen Bereichen als auch bei geschlossenen Bereichen stets so zu verstehen, dass die oberen und unteren Grenzen von dem jeweiligen Bereich mit erfasst sind.

Die Umsetzung erfolgt in einer Ausgestaltung bei einem Gesamtdruck von ca. 1 bis ca. 200 bar, insbesondere bei ca. 40 bar bis ca. 180 bar und ganz besonders bei ca. 60 bar bis ca. 140 bar. Der Gesamtdruck setzt sich dabei aus dem Ammoniakpartialdruck und dem Partialdruck weiterer in der Gasphase enthaltener Gase zusammen. Als solche weiteren Gase kommen beispielsweise Luft oder Stickstoff in Betracht, wobei kleine Mengen dieser Gase gegenüber großen Mengen bevorzugt sind.

In einer Variante erfolgt die Umsetzung unter Einwirkung eines Katalysators, um die Umsatzraten zu erhöhen, die Reaktionstemperaturen auf einem niedrigeren Niveau halten zu können, die Selektivität bezüglich der Produkte zu erhöhen und/oder die Reaktionszeiten zu verkürzen.

Der Katalysator weist in einer alternativen Ausgestaltung ein Metall oder ein Metalloxid auf. Auch Mischungen verschiedener Metalle und/oder Metalloxide sind möglich.

Insbesondere weist der Katalysator ein Metall aus der 8., 9. oder 10. IUPAC-Gruppe (VIII. Nebengruppe) des Periodensystems auf. Dazu gehören unter anderem Eisen, Cobalt, Nickel, Ruthenium, Rhodium, Palladium und Platin.

Die eingesetzte Menge des Katalysators liegt in einer Variante im Bereich von 0,001 bis 20 Mol-%, insbesondere 0,01 bis 10 Mol-%, insbesondere 0,1 bis 1 Mol-% und ganz besonders 0,1 bis 0,5 Mol-%, jeweils bezogen auf die Stoffmenge der umzusetzenden Verbindung.

In einer weiteren Ausgestaltung weist der Katalysator ein Trägermaterial auf. Wird beispielsweise ein poröses Trägermaterial verwendet, lässt sich die Oberfläche des Katalysators erhöhen und die Menge des katalytisch aktiven Metalls oder Metalloxids verringern.

Geignete Trägermaterialien sind beispielsweise Zeolithe, Alumosilikate, Alumophosphate, Metalloxide, Silikate, Schichtsilikate, Aluminiumoxid, Siliziumdioxid sowie Kohlenstoff.

Beispiele für Zeolithe sind Beta-Zeolith (BEA), Y-Zeolith, Faujasit, Mordenit, ZSM-5, Zeolith X, Zeolith A.

Beispiele für Schichtsilikate sind Montmorillonit, Mordenit, Bentonit, Kaolinit, Muskovit, Hectorit, Fluorhectorit, Kanemit, Revdit, Grumantit, Ilerit, Saponit, Beidelit, Nontronit, Stevensit, Laponit, Taneolit, Vermiculit, Halloysit, Volkonskoit, Magadit, Rectorit, Kenyait, Sauconit, Borfluorphlogopite und/oder synthetische Smectite.

Die Katalysatoren können als Pulver-, Formkörper- und Monolithkatalysatoren, letztere beispielsweise auf Wabenkörpern, eingesetzt werden. Für die Umsetzung kommen beispielsweise Festbettreaktoren, Wirbelschichtreaktoren, Rührkesselreaktoren oder Rohrreaktoren zum Einsatz. Die Reaktoren werden in einer Variante für die Umsetzung unter Ammoniakatmosphäre mit Drücken von ca. 1 bis ca. 200 bar bei Temperaturen bis 280°C oder 300 °C beaufschlagt. Temperaturbereiche von ca. 100 °C bis ca. 300 °C, insbesondere ca. 150 °C bis ca. 250 °C und ganz besonders ca. 180 °C bis ca. 240 °C sind bevorzugt.

Die notwendigen Reaktionszeiten liegen in einer Ausgestaltung unter 20 Stunden, insbesondere zwischen ca. 1 Minute und ca. 20 Stunden, insbesondere zwischen ca. 1 Stunde und ca. 10 Stunden, und ganz besonders zwischen ca. 4 Stunden und ca. 8 Stunden.

In einer alternativen Ausgestaltung wird der Katalysator nach erfolgter Umsetzung, das heißt nachdem die Reaktion beendet oder abgebrochen wird, beispielsweise durch Filtration von der Reaktionsmischung getrennt und weiter verarbeitet.

Die Weiterverarbeitung kann beispielsweise ein Erhitzen in einem Lösungsmittel bei ca. 60 °C bis ca. 120 °C, insbesondere ca. 80 °C bis ca. 100 °C, sein. Dadurch ist es möglich, an den Katalysator gebundenes Produkt von dem Katalysator zu lösen und so die Ausbeute der Reaktion zu erhöhen.

Als Lösungsmittel wird in einer Variante Wasser und/oder Aceton verwendet. Beispielsweise ist eine 1:1-Mischung (vol/vol) aus Wasser und Aceton ein geeignetes Lösungsmittel.

Die Ausgangssubstanz ist ein Triazinderivat der allgemeinen Formel (I) oder ein Harnstoff bzw. Harnstoffderivat der allgemeinen Formel (II): 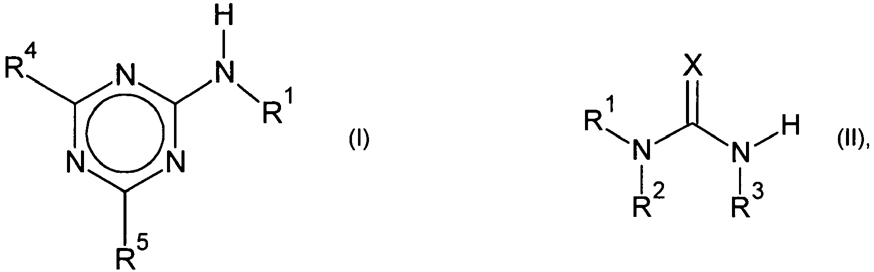 wobei
- **R⁴** und **R⁵** unabhängig voneinander **Q¹** oder einen mit seinem zentralen Stickstoffatom an den Triazinring der Struktur der Formel (I) gebundenen Rest der Formel **R⁶-N-R⁷** oder **R⁸-N-R⁹** bedeuten, wobei
   - **Q¹** ein lineares oder verzweigtes C₁-C₃₀-Alkyl oder einen cyclischen Substituenten in Form eines C₅-C₂₀-Cycloalkyls, eines C₅-C₂₀-Aryls, eines C₁-C₂₀-alkylsubstituierten C₅-C₂₀-Aryls oder eines Imids von cyclischen gesättigten Carbonsäuren, wobei das C₁-C₃₀-Alkyl oder der cyclische Substituent durch ein oder mehrere Sauerstoffatome, Schwefelatome, substituierte Stickstoffatome und/oder durch eine oder mehrere Gruppen des Typs -C(O)O-, -OC(O)-, -C(O)-und/oder -OC(O)O- unterbrochen sein kann, bedeutet,
- **R¹**, **R²**, **R³**, **R⁶**, **R⁷**, **R⁸** und **R⁹** unabhängig voneinander H, lineares oder verzweigtes C₁-C₂₀-Alkyl, C₅-C₂₀-Cycloalkyl, C₅-C₂₀-Aryl, C₁-C₂₀-alkylsubstituiertes C₅-C₂₀-Aryl, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder -OC(O)O- unterbrochen sein und/oder durch eine oder mehrere Hydroxygruppen und/oder Mercaptogruppen funktionalisiert sein kann, bedeuten und
- **X** O oder S bedeutet. Als Alkohol wird eine Verbindung mit der allgemeinen Formel **R¹⁰**-OH verwendet, wobei
- **R¹⁰** lineares oder verzweigtes C₁-C₂₀-Alkyl, C₅-C₂₀-Cycloalkyl, C₁-C₂₀-alkylsubstituiertes C₅-C₂₀-Aryl, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder -OC(O)O- unterbrochen sein und/oder durch eine oder mehrere Hydroxygruppen und/oder Mercaptogruppen funktionalisiert sein kann, bedeutet.

In einer Variante ist mindestens eine Hydroxylgruppe des Alkohols am Alkylrest und nicht am Arylrest gebunden, wenn **R¹⁰** ein C₁-C₂₀-alkylsubstituiertes C₅-C₂₀-Aryl, welches wie zuvor unterbrochen oder substituiert sein kann, bedeutet. Dabei kann es auch vorgesehen sein, dass sämtliche Hydroxylgruppen am Alkylrest und nicht am Arylrest gebunden sind. Auf diese Weise lässt sich die Bildung arylierter Triazin- oder Harnstoffderivate zugunsten der Bildung arylsubstituierter alkylierter Derivate vermeiden.

Sofern Hydroxylgruppen des Alkohols sowohl am Alkylrest als auch am Arylrest vorliegen, wenn **R¹⁰** ein C₁-C₂₀-alkylsubstituiertes C₅-C₂₀-Aryl, welches wie zuvor unterbrochen oder substituiert sein kann, bedeutet, kann über die Wahl geeigneter Reaktionsbedingungen dennoch eine Bildung arylsubstituierter alkylierter Triazin- und Harnstoffderivate gegenüber arylierten Derivaten bevorzugt werden. So reagieren arylische Hydroxylgruppen aufgrund elektronischer Einflüsse des Aromaten grundsätzlich langsamer als Hydroxylgruppen, die an einen Alkylrest gebunden sind.

Beispiele für mögliche Triazinderivate als Ausgangssubstanz sind Melamin, Benzoguanamin, Acetoguanamin, 2,2-Dimethylamino-4,6-diamino-1,3,5-triazin, 2,2-Dibutylamino-4,6-diamino-1,3,5-triazin, 2,4,6-Tris-(2-hydroxyethyl)amino-1,3,5-triazin, 2-Succinimido-4,6-diamino-1,3,5-triazin und 2,4,6-Tris-methylamino-1,3,5-triazin. Beispiele für Harnstoffderivate als Ausgangssubstanz sind Hydroxyethylharnstoff und Ethylenharnstoff. Auch nicht derivatisierter Harnstoff kann als Ausgangssubstanz eingesetzt werden.

Insbesondere werden als Alkohol ein Monoalkohol oder ein Polyalkohol (worunter auch Diole und Oligoole zu verstehen sind) bzw. Mischungen davon eingesetzt.

Beispiele für geeignete Monoalkohole sind Methanol, Ethanol, Propanol, Isopropanol, Butanol, Hexanol, Decanol, Dodecanol, Stearylalkohol, Glykolmonomethylether, Diethylenglycolmonomethylether und Benzylalkohol.

Beispiele für geeignete Polyalkohole sind Ethylenglykol, Diethylenglykol, Glycerol, Trimethylolpropan, Pentaerythrit, Tripropylenglykol, Trisopropanolamin, Triethanolamin, Hexandiol, Butandiol und Glycerolmonostearat.

In einer Variante des Verfahrens reicht es bei kurzkettigen Alkoholen als Edukten für die Derivatisierung, insbesondere Alkylierung, der Ausgangssubstanz aus, den Alkohol im Überschuss einzusetzen, um diesen auch als Lösungsmittel zu nutzen. In einer weiteren Variante werden bei Verwendung von längerkettigen Alkoholen (mehr als 8, 10 oder 12 C-Atome) inerte Lösungsmittel als Lösungsvermittler eingesetzt, um eine bessere Umsetzung zu erreichen. Grundsätzlich können auch Lösungsvermittler bei kurzkettigeren Alkoholen eingesetzt werden, wenn diese beispielsweise stark verzweigt sind und daher eine höhere Viskosität aufweisen. Der Einsatz eines Lösungsvermittlers ist immer dann angebracht, wenn die Mischung ohne Lösungsvermittler nicht mehr rührfähig ist.

Beispiele für derartige Lösungsvermittler sind Tetrahydrofuran, Diethylether, Dimethoxymethan, Dimethoxyethan, Diethoxymethan, Diethoxyethan, Ethylenglykoldiethylether, Ethylenglykoldibutylether, Diethylenglykoldiethylether, Dioxan, Benzen, Toluen, Xylen, Mesitylen, Cumen, Chlorbenzen, Pentan, Hexan, Cyclohexan, Heptan, Octan, Acetonitril, Methylacetat, Ethylacetat, Methylbenzoat, N,N-Dimethylformamid, N,N-Dimethylacetamid, N-Methylpyrrolidon, 1,3-Dimethylimidazolidinon.

In einer Variante ist die gebildete Verbindung ein Triazinderivat der allgemeinen Formel (III) oder Harnstoffderivat der allgemeinen Formel (IV): 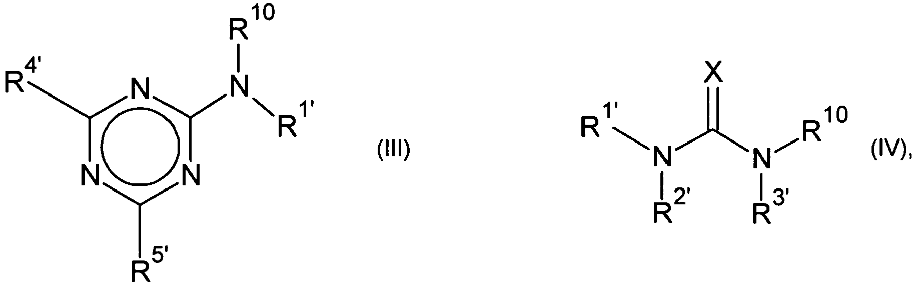 wobei
- **R^{4'}** und **R^{5'}** unabhängig voneinander **Q¹** oder einen mit seinem zentralen Stickstoffatom an den Triazinring der Struktur der Formel (III) gebundenen Rest der Formel **R^{6'}**-N-**R^{7'}** oder **R^{8'}**-N-**R^{9'}** bedeuten, wobei
   - **Q¹** ein lineares oder verzweigtes C₁-C₃₀-Alkyl oder einen cyclischen Substituenten in Form eines C₅-C₂₀-Cycloalkyls, eines C₅-C₂₀-Aryls, eines C₁-C₂₀-alkylsubstituierten C₅-C₂₀-Aryls oder eines Imids von cyclischen gesättigten Carbonsäuren, wobei das C₁-C₃₀-Alkyl oder der cyclische Substituent durch ein oder mehrere Sauerstoffatome, Schwefelatome, substituierte Stickstoffatome und/oder durch eine oder mehrere Gruppen des Typs -C(O)O-, -OC(O)-, -C(O)-und/oder -OC(O)O- unterbrochen sein kann, bedeutet,
- **R^{1'}**, **R^{2'}**, **R^{3'}**, **R^{6'}**, **R^{7'}**, **R^{8'}** und **R^{9'}** unabhängig voneinander
   - **R¹⁰**,
   - H,
   - eine an einen am selben Stickstoffatom des Triazinderivats oder des Harnstoffderivats gebundenen Rest **R¹⁰** gebundene kovalente Bindung, so dass sich eine zyklische Struktur aus dem Stickstoffatom und dem Rest **R¹⁰** ausbildet, oder
   - lineares oder verzweigtes C₁-C₂₀-Alkyl, C₅-C₂₀-Cycloalkyl, C₅-C₂₀-Aryl, C₁-C₂₀-alkylsubstituiertes C₅-C₂₀-Aryl, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder - OC(O)O- unterbrochen sein und/oder durch eine oder mehrere Hydroxygruppen und/oder Mercaptogruppen funktionalisiert sein kann, bedeuten,
- **R¹⁰** lineares oder verzweigtes C₁-C₂₀-Alkyl, C₅-C₂₀-Cycloalkyl, C₁-C₂₀-alkylsubstituiertes C₅-C₂₀-Aryl, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder -OC(O)O- unterbrochen sein und/oder durch eine oder mehrere Hydroxygruppen und/oder Mercaptogruppen funktionalisiert sein kann, bedeutet, wobei durch **R¹⁰** eine zyklische Struktur mit zwei an dasselbe Stickstoffatom der Verbindungen der allgemeinen Formeln (III) oder (IV) gebundenen kovalenten Bindungen ausgebildet sein kann, wobei eine der beiden kovalenten Bindungen durch einen der Reste **R^{1'}**, **R²**, **R^{3'}**, **R^{6'}**, **R**^{**7**'}, **R^{8'}** oder **R^{9'}** bereitgestellt wird und
- **X** O oder S bedeutet.

Eine zyklische Struktur durch den Rest **R¹⁰** bildet sich beispielsweise dann, wenn ein Alkohol eingesetzt wird, der mindestens zwei Hydroxylgruppen trägt, die jeweils mit demselben Stickstoffatom der Ausgangsverbindung reagieren.

In einer Variante des Verfahrens kommt mindestens einem Rest, insbesondere mindestens zwei Resten, insbesondere mindestens drei Resten, insbesondere mindestens vier Resten, insbesondere mindestens fünf Resten der Reste **R^{1'}**, **R^{2'}**, **R^{3'}**, **R^{6'}**, **R^{7'}**, **R^{8'}** und **R^{9'}** nur die Bedeutung des Rests **R¹⁰** und insbesondere nicht die Bedeutung H zu. Dies ist insbesondere dann der Fall, wenn die entsprechenden Reste **R^{1'}**, **R^{2'}**, **R^{3'}**, **R^{6'}**, **R^{7'}**, **R^{8'}** und **R^{9'}** in der Ausgangssubstanz einen Wasserstoffrest bedeutet haben. Mit anderen Worten können unterschiedlich viele Aminogruppen der gebildeten Verbindung (beispielsweise eine, zwei oder drei Aminogruppen) voneinander unabhängig unterschiedlich hoch (beispielsweise einfach oder zweifach) mit dem Rest **R¹⁰** substituiert sein.

Durch geeignete Versuchsparameter können beispielsweise im Wesentlichen reine N,N'-Dialkylverbindungen, N,N',N"-Trialkylverbindungen oder N,N,N',N"-Tetraalkylverbindungen hergestellt werden. Auch können durch geeignete Versuchsparameter im Wesentlichen reine N,N'-Dihydroxyalkylverbindungen, N,N',N"-Trihydroxyalkylverbindungen, N,N',N"-Trihydroxyalkyl-N,N',N"-trialkylverbindungen, N,N,N',N"-Tetrahydroxyalkylverbindungen oder N,N,N',N',N",N"-Hexahydroxyalkylverbindungen hergestellt werden.

In einer Ausgestaltung ist die gebildete Verbindung ausgewählt aus der Gruppe umfassend N-Alkylmelamin, N,N'-Dialkylmelamin, N,N',N"-Trialkylmelamin, N,N,N',N"-Tetraalkylmelamin, N,N,N',N',N"-Pentaalkylmelamin und N,N,N',N',N",N"-Hexaalkylmelamin. In einer weiteren Ausgestaltung ist die gebildete Verbindung ausgewählt aus der Gruppe umfassend N-Alkylharnstoff, N,N'-Dialkylharnstoff, N,N,N'-Trialkylharnstoff und N,N,N',N'-Tetraalkylharnstoff.

In einer weiteren Ausgestaltung ist die gebildete Verbindung ausgewählt aus der Gruppe umfassend N-Alkylthioharnstoff, N,N'-Dialkylthioharnstoff, N,N,N'-Trialkylthioharnstoff und N,N,N',N'-Tetraalkylthioharnstoff.

In einer alternativen Ausgestaltung ist die gebildete Verbindung ausgewählt aus der Gruppe umfassend N-Alkylbenzoguanamin, N,N'-Dialkylbenzoguanamin, N,N,N'-Trialkylbenzoguanamin und N,N,N',N'-Tetraalkylbenzoguanamin.

In einer weiteren alternativen Ausgestaltung ist die gebildete Verbindung ausgewählt aus der Gruppe N-Alkylacetoguanamin, N,N'-Dialkylacetoguanamin und N,N,N'-Trialkylacetoguanamin und N,N,N',N'-Tetraalkylacetoguanamin.

In einer weiteren Ausgestaltung ist die gebildete Verbindung ausgewählt aus der Gruppe umfassend N-(Hydroxyalkyl)-melamin, N,N'-Di-(hydroxyalkyl)-melamin, N,N',N"-Tris-(hydroxyalkyl)-melamin, N,N,N',N"-Tetra-(hydroxyalkyl)-melamin, N,N,N'N',N"-Penta-(hydroxyalkyl)-melamin und N,N,N',N',N",N"-Hexa-(hydroxyalkyl)-melamin.

Als Alkylrest kommen dabei jeweils insbesondere Methyl-, Ethyl-, Butyl und/oder Hexylreste (oder deren Mischungen) in Betracht, aber auch alle anderen für den Rest **R¹⁰** oben angegeben Bedeutungen. Als Hydroxyalkylrest kommen alle Hydroxygruppen tragenden Reste **R¹⁰**, beispielsweise 2-Hydroxyethyl-, Hydroxypropyl- und/oder Hydroxyethoxyethylreste, in Betracht.

Anhand der nachfolgenden Reaktionsgleichungen soll eine beispielhafte Ausgestaltung des beanspruchten Verfahrens näher erläutert werden. Dabei bedeuten "T" eine gegenüber der Raumtemperatur erhöhte Temperatur und "p" einen gegenüber dem Standardluftdruck erhöhten Druck (besondere Parameterausgestaltungen bzw. Reaktionsbedingungen und Bedeutungen der Reste Rⁿ sind weiter oben erläutert):

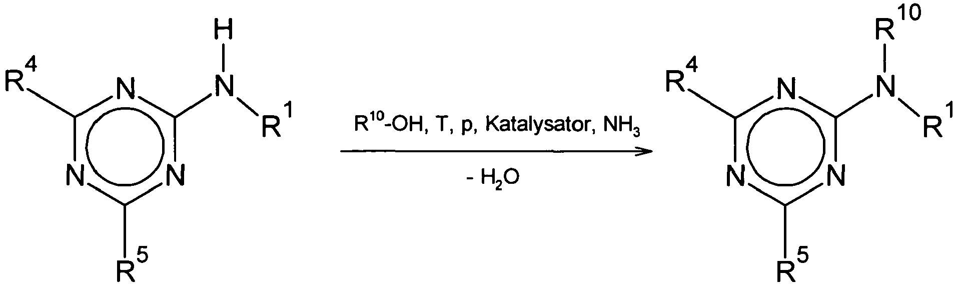

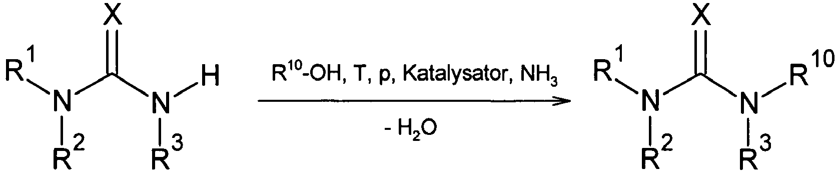

Durch die Umsetzung der Ausgangssubstanz wird ein an den Stickstoff einer Aminogruppe gebundenes Wasserstoffatom durch den Rest **R¹⁰** aus dem eingesetzten Alkohol ersetzt; es erfolgt eine Derivatisierung der Aminogruppe. Nach Abschluss der Reaktion weist die derivatisierte Aminogruppe zumindest einen von einem Wasserstoffatom verschiedenen Substituenten, nämlich **R¹⁰**, auf. Je nach Reaktionsbedingungen können auch weitere Reste **Rⁿ**, die zuvor ein Wasserstoffatom darstellten, durch den Rest **R¹⁰** ersetzt werden. Auf diese Weise lassen sich Verbindungen mit unterschiedlich stark substituierten Aminogruppen herstellen.

Durch die erzielte Reinheit eignen sich die nach diesem Verfahren hergestellten derivatisierten, insbesondere alkylierten, Verbindungen wie beispielsweise alkylierte Aminotriazine und alkylierte Harnstoffe für die Verwendung als Formaldehydharze. Unter dem Begriff "Formaldehydharz" wird dabei ein Harz aus Formaldehyd und der entsprechenden gebildeten Verbindung verstanden. Diese Formaldehydharze weisen spezielle Eigenschaften hinsichtlich Rheologie, Hydrophilie bzw. Lipophilie und Oberflächeneigenschaften auf. Sie eignen sich insbesondere zur Verwendung im Bereich der Laminatbeschichtung der holzverarbeitenden Industrie.

Insbesondere alkylierte Verbindungen wie beispielsweise das symmetrische Trialkylmelamin (N,N',N"-Trialkylmelamin) eignen sich auch als Vernetzer. Weitere Einsatzgebiete der gebildeten Verbindungen, insbesondere der alkylierten Verbindungen wie beispielsweise den alkylierten Aminotriazinen und den alkylierten Harnstoffen, sind der Bereich der Additive zur Plastifizierung, der Bereich der Flammschutzadditive, der Bereich der Comonomere für ein Polyurethan und der Bereich der Agrochemikalien.

Da durch eine Alkylierung (insbesondere dann, wenn längerkettige Alkylreste an die eine Aminogruppe tragende Ausgangssubstanz gebunden werden) die Hydrophobizität der gebildeten Verbindung gegenüber der Ausgangsverbindung erhöht wird, eignen sich die mit dem beanspruchten Verfahren hergestellten Verbindungen dazu, in einer Mischung mit einem Polyolefin, insbesondere einem Polyethylen (Polyethen) oder Polypropylen (Polypropen), eingesetzt zu werden. Auf diese Weise lassen sich beispielsweise die Flammschutzeigenschaften oder Oberflächeneigenschaften eines aus der Mischung des Polyolefins und der gebildeten Verbindung bestehenden Gegenstands gegenüber den entsprechenden Eigenschaften eines aus einem unmodifizierten Polyolefin bestehenden Gegenstands verbessern.

Weitere Details der Erfindung werden anhand der nachfolgenden Beispiele näher erläutert, wobei das zunächst folgende Vergleichsbeispiel 1 ein Verfahren gemäß dem Stand der Technik mit niedriger Selektivität bezüglich der gebildeten Produkte wiedergibt. Sofern nicht explizit etwas anderes angegeben wird, sind alle Prozentangaben in den Beispielen wie auch in den übrigen Teilen der Beschreibung und den Ansprüchen als Massenprozent zu verstehen.

### Herstellung von N,N'-Dimethylmelamin

Vergleichsbeispiel 1:
In einem 500ml Rührautoklav werden 5,0 g Melamin, 104 g Methanol und 10,1 g eines Ni/Y-Zeolith Katalysators intensiv gemischt, damit der Katalysator nicht zu Boden sinkt. Nach Verschließen des Autoklaven wird auf 200°C aufgeheizt. Dabei stellt sich ein Druck von 40 bar ein. Nach 6 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton/Wasser-Gemisch (1:1) bei 80°C ausgekocht. Das Filtrat und die Waschlösung wurden vereint und bis zur Trockne eingeengt. Das entstehende Produkt wird im Vakuumtrockenschrank bei 40°C getrocknet. Auf diese Weise wurden 4,9 g Produkt isoliert, was einer Ausbeute von 81% bezogen auf eingesetztes Melamin entspricht. Eine Bestimmung der Zusammensetzung des Produkts mittels quantitativer HPLC ergab 25,7% Melamin, 10,1% N-Methylmelamin, 1,2% N,N-Dimethylmelamin, 1,6% N,N'-Dimethylmelamin, 8,5% O-Methylammelin, 9,3% O,O'-Dimethylammelid und 43,6% Trimethylcyanurat.

### Beispiel 1a:

In einem 500ml Rührautoklav werden 5,0 g Melamin, 205 g Methanol und 10,2 g eines Ni/Y-Zeolith Katalysators intensiv gemischt, damit der Katalysator nicht zu Boden sinkt. Nach Verschließen des Autoklaven werden 105 g Ammoniak in den Reaktor gedrückt und auf 210°C aufgeheizt. Dabei stellt sich ein Druck von 130 bar ein. Nach 3 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton/Wasser-Gemisch (1:1) bei 80°C ausgekocht. Das Filtrat und die Waschlösung wurden vereint und bis zur Trockne eingeengt. Das entstehende Produkt wird im Vakuumtrockenschrank bei 40°C getrocknet. Auf diese Weise wurden 5,7 g Produkt isoliert. Eine Bestimmung der Zusammensetzung des Produkts mittels quantitativer HPLC ergab 93,6 % N,N'-Dimethylmelamin, 5,0 % N-Methylmelamin und 1,3 % N,N',N"-Trimethylmelamin sowie 0,1 % Trimethylcyanurat.

### Beispiel 1b:

In einem 500ml Rührautoklav werden 5,4 g Melamin, 109 g Methanol und 10,3 g eines Ru/Mordenit Katalysators intensiv gemischt, damit der Katalysator nicht zu Boden sinkt. Nach Verschließen des Autoklaven werden 105 g Ammoniak in den Reaktor gedrückt und auf 200°C aufgeheizt. Dabei stellt sich ein Druck von 120 bar ein. Nach 6 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton/Wasser-Gemisch (1:1) bei 80°C ausgekocht. Das Filtrat und die Waschlösung wurden vereint und bis zur Trockne eingeengt. Das entstehende Produkt wird im Vakuumtrockenschrank bei 40°C getrocknet. Auf diese Weise wurden 6,6 g Produkt isoliert. Eine Bestimmung der Zusammensetzung des Produkts mittels quantitativer HPLC ergab 62 % N,N'-Dimethylmelamin, 2,1 % N-Methylmelamin und 35,9 % N,N',N"-Trimethylmelamin.

### Herstellung von N,N',N"-Trimethylmelamin

### Beispiel 2a:

In einem 500ml Rührautoklav werden 5,3 g Melamin, 107 g Methanol und 10,4 g eines Ru/BEA Katalysators intensiv gemischt, damit der Katalysator nicht zu Boden sinkt. Nach Verschließen des Autoklaven werden 100 g Ammoniak in den Reaktor gedrückt und auf 230°C aufgeheizt. Dabei stellt sich ein Druck von 140 bar ein. Nach 4 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton/Wasser-Gemisch (1:1) bei 80°C ausgekocht. Das Filtrat und die Waschlösung wurden vereint und bis zur Trockne eingeengt. Das entstehende Produkt wird im Vakuumtrockenschrank bei 40°C getrocknet. Auf diese Weise wurden 6,5 g Produkt isoliert. Eine Bestimmung der Zusammensetzung des Produkts mittels quantitativer HPLC ergab 99% N,N',N"-Trimethylmelamin sowie 1 % N,N,N',N"-Tetramethylmelamin.

### Beispiel 2b:

In einem 500ml Rührautoklav werden 5,3 g Melamin, 120 g einer 15%igen ammoniakalischen Methanollösung und 10,4 g eines Ru/BEA Katalysators intensiv gemischt, damit der Katalysator nicht zu Boden sinkt. Nach Verschließen des Autoklaven wird auf 230°C aufgeheizt. Dabei stellt sich ein Druck von 70 bar ein. Nach 4 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton/Wasser-Gemisch (1:1) bei 80°C ausgekocht. Das Filtrat und die Waschlösung wurden vereint und bis zur Trockne eingeengt. Das entstehende Produkt wird im Vakuumtrockenschrank bei 40°C getrocknet. Auf diese Weise wurden 6,7 g Produkt isoliert. Eine Bestimmung der Zusammensetzung des Produkts mittels quantitativer HPLC ergab 97,6% N,N',N"-Trimethylmelamin sowie 2,4% N,N,N',N"-Tetramethylmelamin.

### Herstellung von N,N-N',N"-Tetramethylmelamin

### Beispiel 3a:

In einem 500ml Rührautoklav werden 5,4 g Melamin, 208 g Methanol und 10,3 g eines Ru/BEA Katalysators intensiv gemischt, damit der Katalysator nicht zu Boden sinkt. Nach Verschließen des Autoklaven werden 55,5 g Ammoniak in den Reaktor gedrückt und auf 230°C aufgeheizt. Dabei stellt sich ein Druck von 140 bar ein. Nach 8 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton/Wasser-Gemisch (1:1) bei 80°C ausgekocht. Das Filtrat und die Waschlösung werden vereint und bis zur Trockne eingeengt. Das entstehende Produkt wird im Vakuumtrockenschrank bei 40°C getrocknet. Auf diese Weise wurden 6,9 g Produkt isoliert. Eine Bestimmung der Zusammensetzung des Produkts mittels quantitativer HPLC ergab 72,3% N,N,N',N"-Tetramethylmelamin, 25,8% N,N',N"-Trimethylmelamin sowie 1,1% N,N,N'-Trimethylmelamin und 0,8% N,N,N',N'-Tetramethylmelamin.

### Beispiel 3b:

In einem 500ml Rührautoklav werden 5,1 g eines Gemisches aus 30% N-Methylmelamin, 34% N,N'-Dimethylmelamin und 36% N,N'N"-Trimethylmelamin, 202 g Methanol und 10,4 g eines Ru/BEA Katalysators intensiv gemischt, damit der Katalysator nicht zu Boden sinkt. Nach Verschließen des Autoklaven werden 60 g Ammoniak in den Reaktor gedrückt und auf 240°C aufgeheizt. Dabei stellt sich ein Druck von 135 bar ein. Nach 4 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton/Wasser-Gemisch (1:1) bei 80°C ausgekocht. Das Filtrat und die Waschlösung werden vereint und bis zur Trockne eingeengt. Das entstehende Produkt wird im Vakuumtrockenschrank bei 40°C getrocknet. Auf diese Weise wurden 5,4 g Produkt isoliert. Eine Bestimmung der Zusammensetzung des Produkts mittels quantitativer HPLC ergab 84,2% N,N,N',N"-Tetramethylmelamin, 15,6% N,N',N"-Trimethylmelamin sowie 0,2% N,N,N',N',N"-Pentamethylmelamin.

### Herstellung von N,N',N"-Triethylmelamin

### Beispiel 4:

In einem 500ml Rührautoklav werden 5,1 g Melamin, 303 g Ethanol und 10,2 g eines Ni/NiO Katalysators intensiv gemischt, damit der Katalysator nicht zu Boden sinkt. Nach Verschließen des Autoklaven werden 103,8 g Ammoniak in den Reaktor gedrückt und auf 240°C aufgeheizt. Dabei stellt sich ein Druck von 155 bar ein. Nach 4 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton/Wasser-Gemisch (1:1) bei 80°C ausgekocht. Das Filtrat und die Waschlösung werden vereint und bis zur Trockne eingeengt. Das entstehende Produkt wird im Vakuumtrockenschrank bei 40°C getrocknet. Auf diese Weise wurden 7,8 g Produkt isoliert. Eine Bestimmung der Zusammensetzung des Produkts mittels quantitativer HPLC ergab 94,6% N,N',N"-Triethylmelamin und 5,4% N,N,N',N"-Tetraethylmelamin.

### Methylierung von Harnstoff

### Beispiel 5:

In einem 500ml Rührautoklav werden 3,0 g Harnstoff, 80 g Methanol und 5 g eines Ru/BEA Katalysators intensiv gemischt, damit der Katalysator nicht zu Boden sinkt. Nach Verschließen des Autoklaven werden 100 g Ammoniak in den Reaktor gedrückt und auf 200°C aufgeheizt. Nach 4 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton/Wasser-Gemisch (1:1) bei 80°C ausgekocht. Das Filtrat und die Waschlösung wurden vereint und bis zur Trockne eingeengt. Das entstehende Produkt wird im Vakuumtrockenschrank bei 40°C getrocknet. Auf diese Weise wurden 3,5 g Produkt isoliert. Eine Bestimmung der Zusammensetzung des Produkts mittels quantitativer HPLC ergab 84,6% N,N'-Dimethylharnstoff, 3,4% N-Monomethylharnstoff und 12% N,N,N'-Trimethylharnstoff.

### Methylierung von Benzoguanamin

### Beispiel 6:

In einem 500ml Rührautoklav werden 6,0 g Benzoguanamin, 110 g Methanol und 9,5 g eines Ni/NiO Katalysators intensiv gemischt, damit der Katalysator nicht zu Boden sinkt. Nach Verschließen des Autoklaven werden 80 g Ammoniak in den Reaktor gedrückt und auf 230°C aufgeheizt. Nach 4 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton ausgekocht. Das Filtrat und die Waschlösung wurden vereint und bis zur Trockne eingeengt. Das entstehende Produkt wird im Vakuumtrockenschrank bei 40°C getrocknet. Auf diese Weise wurden 6,5 g Produkt isoliert. Eine Bestimmung der Zusammensetzung des Produkts mittels quantitativer HPLC ergab 78,5% N,N'-Dimethylbenzoguanamin, 6,7% N-Monomethylbenzoguanamin und 14,8% N,N,N'-Trimethylbenzoguanamin.

### Ethylierung von Acetoguanamin

### Beispiel 7:

In einem 500ml Rührautoklav werden 5,6 g Acetoguanamin, 290 g Ethanol und 10,2 g eines Ru/TiO₂ Katalysators intensiv gemischt, damit der Katalysator nicht zu Boden sinkt. Nach Verschließen des Autoklaven werden 100 g Ammoniak in den Reaktor gedrückt und auf 210°C aufgeheizt. Nach 3 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton/Wasser-Gemisch (1:1) bei 80°C ausgekocht. Das Filtrat und die Waschlösung werden vereint und bis zur Trockne eingeengt. Das entstehende Produkt wird im Vakuumtrockenschrank bei 40°C getrocknet. Auf diese Weise wurden 6,5 g Produkt isoliert. Eine Bestimmung der Zusammensetzung des Produkts mittels quantitativer HPLC ergab 74,8% N,N'-Diethylacetoguanamin und 25,2 % N,N,N'-Triethylacetoguanamin.

### Herstellung von Hexylmelamin

### Beispiel 8:

In einem 1000ml Rührautoklav werden 5,1 g Melamin, 500 g Hexanol und 10,2 g eines Ni/NiO Katalysators intensiv gemischt, damit der Katalysator nicht zu Boden sinkt. Nach Verschließen des Autoklaven werden 103,8 g Ammoniak in den Reaktor gedrückt und auf 250°C aufgeheizt. Nach 8 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton bei 80°C ausgekocht. Das Filtrat und die Waschlösung werden vereint und bis zur Trockne eingeengt. Das entstehende Produkt wird im Vakuumtrockenschrank bei 40°C getrocknet. Auf diese Weise wurden 7,8 g Produkt isoliert. Eine Bestimmung der Zusammensetzung des Produkts mittels quantitativer HPLC ergab 55% N,N',N"-Trihexylmelamin, 27% N,N'-Dihexylmelamin und 15% N-Monohexylmelamin sowie 3% Melamin.

### Herstellung von Tris-(2-hydroxyethyl)-melamin

### Beispiel 9:

In einem 1000ml Rührautoklav werden 5,0 g Melamin, 400 g Ethylenglycol und 9,5 g eines Ru/Mordenit Katalysators intensiv gemischt, damit der Katalysator nicht zu Boden sinkt. Nach Verschließen des Autoklaven werden 120 g Ammoniak in den Reaktor gedrückt und auf 210°C aufgeheizt. Nach 6 Stunden Reaktionszeit wird die Reaktion abgebrochen und der Autoklav abgekühlt und entspannt. Die erkaltete Lösung wird über einen Filter vom Katalysator getrennt. Der Katalysator wird mit Wasser bei 100°C ausgekocht. Das Filtrat und die Waschlösung werden vereint und bis zur Trockne eingeengt. Das entstehende Produkt wird im Vakuumtrockenschrank bei 40°C getrocknet. Auf diese Weise wurden 8,1 g Produkt isoliert. Eine Bestimmung der Zusammensetzung des Produkts mittels quantitativer HPLC ergab 64% N,N',N"-Tris-(2-hydroxyethyl)melamin, 16% N,N'-Di-(2-hydroxyethyl)melamin und 6% N-Mono-(2-hydroxyethyl)melamin sowie 14% N,N,N',N"-Tetra-(2-hydroxyethyl)melamin.

### Herstellung von N,N',N"-Tris-(2-hydroxyethyl)-N,N',N"-trimethylmelamin

### Beispiel 10:

In einem 1000ml Rührautoklav werden 5,5 g N,N',N"-Tris-(2-hydroxyethyl)-melamin, 250 g Methanol und 10,2 g eines Ru/Al₂O₃ Katalysators intensiv gemischt, damit der Katalysator nicht zu Boden sinkt. Nach Verschließen des Autoklaven werden 160 g Ammoniak in den Reaktor gedrückt und auf 200°C aufgeheizt. Nach 6 Stunden Reaktionszeit wird die Reaktion abgebrochen und der Autoklav abgekühlt und entspannt. Die erkaltete Lösung wird über einen Filter vom Katalysator getrennt. Der Katalysator wird mit Aceton/Wasser-Gemisch (1:1) bei 80°C ausgekocht. Das Filtrat und die Waschlösung werden vereint und bis zur Trockne eingeengt. Das entstehende Produkt wird im Vakuumtrockenschrank bei 40°C getrocknet. Auf diese Weise wurden 5,9 g Produkt isoliert. Eine Bestimmung der Zusammensetzung des Produkts mittels quantitativer HPLC ergab 85% N,N',N"-Tris-(2-hydroxyethyl)-N,N',N"-trimethylmelamin, 12% N,N',N"-Tris-(2-hydroxyethyl)-N,N'-dimethylmelamin und 3% N,N',N"-Tris-(2-hydroxyethyl)-N-monomethylmelamin.

### Herstellung von Hexa-(2-hydroxyethyl)-melamin

### Beispiel 11:

In einem 1000ml Rührautoklav werden 5,0 g Melamin, 500 g Ethylenglycol und 10,5 g eines Ru/BEA Katalysators intensiv gemischt, damit der Katalysator nicht zu Boden sinkt. Nach Verschließen des Autoklaven werden 120 g Ammoniak in den Reaktor gedrückt und auf 220°C aufgeheizt. Nach 14 Stunden Reaktionszeit wird die Reaktion abgebrochen und der Autoklav abgekühlt und entspannt. Die erkaltete Lösung wird über einen Filter vom Katalysator getrennt. Der Katalysator wird mit Wasser bei 100°C ausgekocht. Das Filtrat und die Waschlösung werden vereint und bis zur Trockne eingeengt. Das entstehende Produkt wird aus Butanol umkristallisiert und im Vakuumtrockenschrank bei 40°C getrocknet. Auf diese Weise wurden 11,3 g Produkt isoliert. Eine Bestimmung der Zusammensetzung des Produkts mittels quantitativer HPLC ergab 53% N,N,N',N',N",N"-Hexa-(2-hydroxyethyl)melamin, 18% N,N,N',N',N"-Penta-(2-hydroxyethyl)melamin, 16% N,N,N',N"-Tetra-(2-hydroxyethyl)melamin, 9% N,N',N"-Tris-(2-hydroxyethyl)-melamin, und 4% N,N'-Di-(2-hydroxyethyl)-melamin.

## Patentansprüche

1. Verfahren zur Herstellung einer Verbindung mit mindestens einer zumindest einfach substituierten Aminogruppe, **dadurch gekennzeichnet, dass** eine mindestens eine Aminogruppe tragende Ausgangssubstanz in Form eines Triazinderivates der allgemeinen Formel (I) oder eines Harnstoff bzw. Harnstoffderivates der allgemeinen Formel (II) 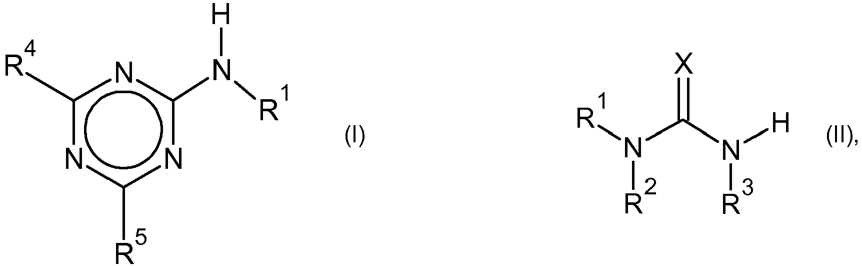 wobei
• **R⁴** und **R⁵** unabhängig voneinander **Q¹** oder einen mit seinem zentralen Stickstoffatom an den Triazinring der Struktur der Formel (I) gebundenen Rest der Formel **R⁶**-N-**R⁷** oder **R⁸-**N**-R⁹** bedeuten, wobei
- **Q¹** ein lineares oder verzweigtes C₁-C₃₀-Alkyl oder einen cyclischen Substituenten in Form eines C₅-C₂₀-Cycloalkyls, eines C₅-C₂₀-Aryls, eines C₁-C₂₀-alkylsubstituierten C₅-C₂₀-Aryls oder eines Imids von cyclischen gesättigten Carbonsäuren, wobei das C₁-C₃₀-Alkyl oder der cyclische Substituent durch ein oder mehrere Sauerstoffatome, Schwefelatome, substituierte Stickstoffatome und/oder durch eine oder mehrere Gruppen des Typs -C(O)O-, -OC(O)-, -C(O)-und/oder -OC(O)O- unterbrochen sein kann, bedeutet,
• **R¹**, **R²**, **R³**, **R⁶**, **R⁷**, **R⁸** und **R⁹** unabhängig voneinander H, lineares oder verzweigtes C₁-C₂₀-Alkyl, C₅-C₂₀-Cycloalkyl, C₅-C₂₀-Aryl, C₁-C₂₀-alkylsubstituiertes C₅-C₂₀-Aryl, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder -OC(O)O- unterbrochen sein und/oder durch eine oder mehrere Hydroxygruppen und/oder Mercaptogruppen funktionalisiert sein kann, bedeuten und
• **X** O oder S bedeutet.
mit einem Alkohol der allgemeinen Formel **R¹⁰**-OH, wobei **R¹⁰** lineares oder verzweigtes C₁-C₂₀-Alkyl, C₅-C₂₀-Cycloalkyl, oder C₁-C₂₀-alkylsubstituiertes C₅-C₂₀-Aryl, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder -OC(O)O- unterbrochen sein und/oder durch eine oder mehrere Hydroxygruppen und/oder Mercaptogruppen funktionalisiert sein kann, bedeutet,
in einer Reaktionsmischung in Gegenwart von Ammoniak umgesetzt wird, wobei das Stoffmengenverhältnis von Ammoniak zu Alkohol 0,1 bis 2, insbesondere 0,5 bis 1,5 und ganz besonders 0,8 bis 1,3 beträgt.

2. Verfahren nach Anspruch 1, **dadurch gekennzeichnet, dass** die Umsetzung bei einem Gesamtdruck von 1 bis 200 bar, insbesondere 40 bis 180 bar und ganz besonders 60 bis 140 bar erfolgt.

3. Verfahren nach Anspruch 1 oder 2, **dadurch gekennzeichnet, dass** die Reaktionsmischung einen Katalysator, insbesondere einen Katalysator aus Metall und/oder Metalloxid, aufweist.

4. Verfahren nach einem der vorhergehenden Ansprüche, **dadurch gekennzeichnet, dass** die Umsetzung bei einer Temperatur von 100 °C bis 300 °C, insbesondere 150 °C bis 250 °C, insbesondere 180 °C bis 240 °C erfolgt.

5. Verfahren nach einem der vorhergehenden Ansprüche, **dadurch gekennzeichnet, dass** die Umsetzung während einer Reaktionsdauer von 1 Minute bis 20 Stunden, insbesondere 1 Stunde bis 10 Stunden und ganz besonders 4 Stunden bis 8 Stunden erfolgt.

6. Verfahren nach einem der vorhergehenden Ansprüche, **dadurch gekennzeichnet, dass** mindestens eine Hydroxylgruppe des Alkohols am Alkylrest und nicht am Arylrest vorliegt, wenn **R¹⁰** ein C₁-C₂₀-alkylsubstituiertes C₅-C₂₀-Aryl bedeutet.

7. Verfahren nach einem der vorhergehenden Ansprüche, **dadurch gekennzeichnet, dass** der Alkohol ein Monoalkohol oder ein Polyalkohol ist.

8. Verfahren nach einem der vorhergehenden Ansprüche, **dadurch gekennzeichnet, dass** die gebildete Verbindung ein Triazinderivat der allgemeinen Formel (III) oder Harnstoffderivat der allgemeinen Formel (IV) ist: 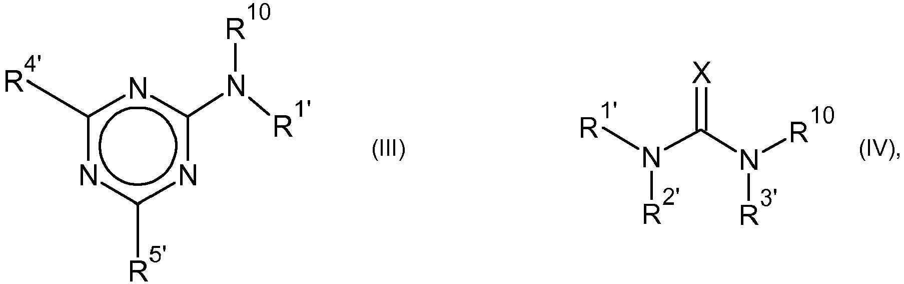 wobei
• **R^{4'}** und **R^{5'}** unabhängig voneinander **Q¹** oder einen mit seinem zentralen Stickstoffatom an den Triazinring der Struktur der Formel (III) gebundenen Rest der Formel **R^{6'}**-N-**R^{7'}** oder **R^{8'}**-N-**R^{9'}** bedeuten, wobei
- **Q¹** ein lineares oder verzweigtes C₁-C₃₀-Alkyl oder einen cyclischen Substituenten in Form eines C₅-C₂₀-Cycloalkyls, eines C₅-C₂₀-Aryls, eines C₁-C₂₀-alkylsubstituierten C₅-C₂₀-Aryls oder eines Imids von cyclischen gesättigten Carbonsäuren, wobei das C₁-C₃₀-Alkyl oder der cyclische Substituent durch ein oder mehrere Sauerstoffatome, Schwefelatome, substituierte Stickstoffatome und/oder durch eine oder mehrere Gruppen des Typs -C(O)O-, -OC(O)-, -C(O)-und/oder -OC(O)O- unterbrochen sein kann, bedeutet,
• **R^{1'}**, **R²**, **R^{3'}**, **R^{6'}**, **R^{7'}**, **R^{8'}** und **R^{9'}** unabhängig voneinander
- **R¹⁰** ,
- H,
- eine an einen am selben Stickstoffatom des Triazinderivats oder des Harnstoffderivats gebundenen Rest **R¹⁰** gebundene kovalente Bindung, so dass sich eine zyklische Struktur aus dem Stickstoffatom und dem Rest **R¹⁰** ausbildet, oder
- lineares oder verzweigtes C₁-C₂₀-Alkyl, C₅-C₂₀-Cycloalkyl, C₅-C₂₀-Aryl, C₁-C₂₀-alkylsubstituiertes C₅-C₂₀-Aryl, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder - OC(O)O- unterbrochen sein und/oder durch eine oder mehrere Hydroxygruppen und/oder Mercaptogruppen funktionalisiert sein kann, bedeuten,
• **R¹⁰** lineares oder verzweigtes C₁-C₂₀-Alkyl, C₅-C₂₀-Cycloalkyl oder C₁-C₂₀-alkylsubstituiertes C₅-C₂₀-Aryl, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder -OC(O)O- unterbrochen sein und/oder durch eine oder mehrere Hydroxygruppen und/oder Mercaptogruppen funktionalisiert sein kann, bedeutet, wobei durch **R¹⁰** eine zyklische Struktur mit zwei an dasselbe Stickstoffatom der Verbindungen der allgemeinen Formeln (III) oder (IV) gebundenen kovalenten Bindungen ausgebildet sein kann, wobei eine der beiden kovalenten Bindungen durch einen der Reste **R^{1'}**, **R², R^{3'}**, **R^{6'}**, **R^{7'}**, **R^{8'}** oder **R^{9'}** bereitgestellt wird und
• **X** O der S bedeutet.

9. Verfahren nach Anspruch 8, **dadurch gekennzeichnet, dass** mindestens einem Rest, insbesondere mindestens zwei Resten, insbesondere mindestens drei Resten, insbesondere mindestens vier Resten, insbesondere mindestens fünf Resten der Reste **R^{1'}**, **R^{2'}**, **R^{3'}**, **R^{6'}**, **R^{7'}**, **R^{8'}** und **R^{9'}** die Bedeutung des Rests **R¹⁰** zukommt.

10. Verfahren nach einem der vorhergehenden Ansprüche, **dadurch gekennzeichnet, dass** die gebildete Verbindung ausgewählt ist aus der Gruppe umfassend N,N'-Dialkylmelamin, N,N',N"-Trialkylmelamin und N,N,N',N"-Tetraalkylmelamin.

11. Verfahren nach einem der vorhergehenden Ansprüche, **dadurch gekennzeichnet, dass** die gebildete Verbindung ausgewählt ist aus der Gruppe umfassend N-Monoalkylharnstoff, N,N'-Dialkylharnstoff und N,N,N'-Trialkylharnstoff.

12. Verfahren nach einem der vorhergehenden Ansprüche, **dadurch gekennzeichnet, dass** die gebildete Verbindung ausgewählt ist aus der Gruppe umfassend N-Monoalkylthioharnstoff, N,N'-Dialkylthioharnstoff und N,N,N'-Trialkylthioharnstoff.

13. Verfahren nach einem der vorhergehenden Ansprüche, **dadurch gekennzeichnet, dass** die gebildete Verbindung ausgewählt ist aus der Gruppe umfassend N,N'-Dialkylbenzoguanamin, N,N,N'-Trialkylbenzoguanamin, N,N'-Dialkylacetoguanamin und N,N,N'-Trialkylacetoguanamin.

14. Verfahren nach einem der vorhergehenden Ansprüche, **dadurch gekennzeichnet, dass** die gebildete Verbindung ausgewählt ist aus der Gruppe umfassend N,N'-Di-(hydroxyalkyl)-melamin, N,N',N"-Tris-(hydroxyalkyl)-melamin, N,N,N',N"-Tetra-(hydroxyalkyl)-melamin, N,N,N'N',N"-Penta-(hydroxyalkyl)-melamin und N,N,N',N',N",N"-Hexa-(hydroxyalkyl)-melamin.

## Claims

1. Method for producing at least one compound having at least one at least monosubstituted amino group, **characterized in that** a starting substance having at least one amino group in form of a triazine derivative of the general formula (I) or a urea or urea derivative of the general formula (II): 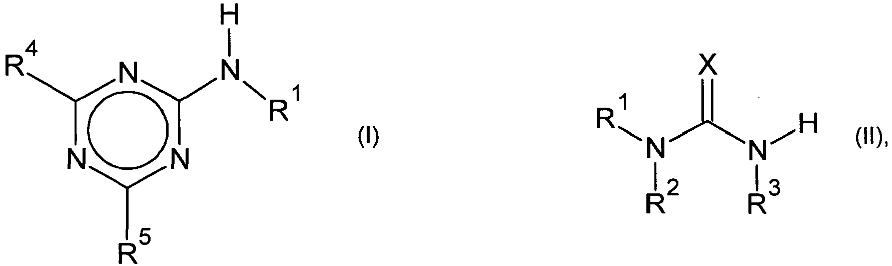 whereby
- R⁴ and R⁵ mean independently from each other Q¹ or a moiety of the formula R⁶-N-R⁷ or R⁸-N-R⁹ bound with its central nitrogen atom to the triazine ring of the structure of formula (I), whereat
- Q¹ means a linear or branched C₁-C₃₀-alkyl or a cyclic substituent in form of a C₅-C₂₀-cyclo alkyl, a C₅-C₂₀-aryl, a C₁-C₂₀-alkylsubstituted C₅-C₂₀-aryl or an amide of a cyclic unsaturated carboxylic acid, whereat the C₁-C₃₀-alkyl or the cyclic substituent can be interrupted by one or multiple oxygen atoms, sulphur atoms, substituted nitrogen atoms and/or by one or multiple groups of the type -C(O)O-,-OC(O)-,-C(O)- and/or-OC(O)O-,
- R¹, R², R³, R⁶, R⁷, R⁸ and R⁹ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl, which in each case can be interrupted by one or multiple oxygen atoms, sulphur atoms and/or substituted nitrogen atoms and/or by one or multiple groups of the type -C(O)O-, - OC(O)-, -C(O)- and/or -OC(O) O- and/or can be functionalized by one or multiple hydroxyl groups and/or mercapto groups, and
- X means O or S.
is reacted with an alcohol of the general formula R¹⁰-OH, whereby R¹⁰ means a linear or branched C₁-C₂₀-alkyl, C₅-C₂₀-cycloalkyl, or C₁-C₂₀-alkylsubstituted C₅-C₂₀-aryl, which can be in each case interrupted by one or multiple oxygen atoms, sulphur atoms, substituted nitrogen atoms and/or by one or multiple groups of the type -C(O)O-, -OC(O)-, -C(O)-and/or -OC(O)O- and/or can be functionalized by one or multiple hydroxyl groups and/or mercapto groups,
in a reaction mixture in the presence of ammonia, whereby the molar ratio of ammonia to alcohol is 0.1 to 2, in particular 0.5 to 1.5 and specifically in particular 0.8 to 1.3.

2. Method according to claim 1, **characterized in that** the conversion occurs at a total pressure of 1 to 200 bar, in particular 40 to 180 bar and in particular 60 to 140 bar.

3. Method according to claim 1 or 2, **characterized in that** the reaction mixture contains a catalyst, in particular a catalyst in form of a metal and/or a metal oxide.

4. Method according to one of the preceding claims, **characterized in that** the conversion occurs at a temperature of 100°C to 300°C, in particular 150°C to 250°C, in particular 180°C to 240°C.

5. Method according to one of the preceding claims, **characterized in that** the conversion occurs during a reaction time of 1 minute to 20 hours, in particular 1 hour to 10 hours and specifically in particular 4 hours to 8 hours.

6. Method according to one of the preceding claims, **characterized in that** that at least one hydroxyl group of the alcohol is present on the alkyl moiety and not on the aryl moiety if R¹⁰ means a C₁-C₂₀-alkyl substituted C₅-C₂₀-aryl.

7. Method according to one of the preceding claims, **characterized in that** the alcohol is a monoalcohol or a polyalcohol.

8. Method according to one of the preceding claims, **characterized in that** the produced compound is a triazine derivative of the general formula (III) or a urea derivative of the general formula (IV): 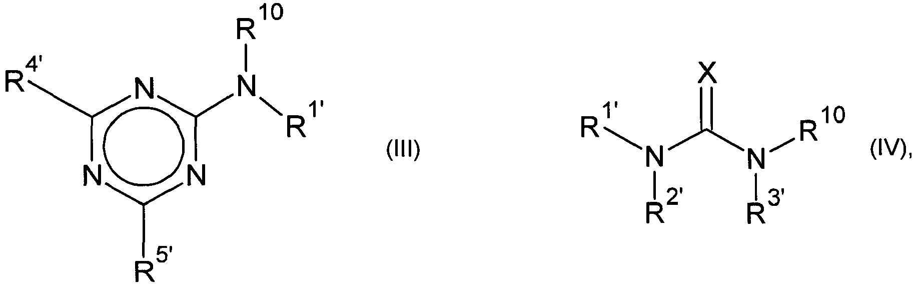 whereby
- R^{4'} and R^{5'} mean independently from each other Q¹ or a moiety of the formula R^{6'}-N-R^{7'}- or R^{8'}-N-R^{9'} being bound with its central nitrogen atom to the triazine ring of the structure of the formula (III), whereat
- Q¹ means a linear or branched C₁-C₃₀-alkyl or a cyclic substituent in form of a C₅-C₂₀-cycloalkyl, a C₅-C₂₀-aryl, a C₁-C₂₀-alkyl substituted C₅-C₂₀-aryl or an amide of a cyclic unsaturated carboxylic acid, whereat the C₁-C₃₀-alkyl or the cyclic substituent can be interrupted by one or multiple oxygen atoms, sulphur atoms, substituted nitrogen atoms and/or by one or multiple groups of the type -C(O)O-,-OC(O)-,-C(O)- and/or -OC(O)O-,
- R^{1'}, R^{2'}, R^{3'}, R^{6'}, R^{7'}, R^{8'} and R^{9'} mean independently from each other,
- R¹⁰,
- H
- a covalent bond bound to the moiety R¹⁰ being bound to the same nitrogen atom of the triazine derivative or urea derivative so that a cyclic structure is formed from the nitrogen atom and the moiety R¹⁰, or
- linear or branched C₁-C₂₀-alkyl, C₅-C₂₀-cycloalkyl, C₅-C₂₀-aryl, C₁-C₂₀-alkyl substituted C₅-C₂₀-aryl, which in each case can be interrupted by one or multiple oxygen atoms, sulphur atoms and/or substituted nitrogen atoms and/or by one or multiple groups of the type -C(O)O-, -OC(O)-, -C(O)- and/or -OC(O)O- and/or can be functionalized by one or multiple hydroxy groups and/or mercapto groups,
- R¹⁰ means a linear or branched C₁-C₂₀-alkyl, C₅-C₂₀-cycloalkyl or C₁-C₂₀-alkyl substituted C₅-C₂₀-aryl, which in each case can be interrupted by one or multiple oxygen atoms, sulphur atoms, substituted nitrogen atoms and/or by one or multiple groups of the type -C(O)O-, -OC(O)-, -C(O)- and/or -OC(O)O-, and/or can be functionalized by one or multiple hydroxyl groups and/or mercapto groups, whereby R¹⁰ can form a cyclic structure with two covalent bonds bonded to the same nitrogen atom of the compounds of the general formula (III) or (IV), whereat one of the two covalent bonds is provided by one of the moieties R^{1'}, R^{2'}, R^{3'}, R^{6'}, R^{7'}, R^{8'} or R^{9'},
- X means O or S.

9. Method according to claim 8, **characterized in that** the at least one moiety, in particular at least two moieties, in particular at least three moieties, in particular at least four moieties, in particular at least five moieties of the moieties R^{1'}, R^{2'}, R^{3'}, R^{6'}, R^{7'}, R^{8'} and R^{9'} have the meaning of the moiety R¹⁰.

10. Method according to one of the preceding claims, **characterized in that** that the formed compound is selected from the group comprising N,N'-dialkyl melamine, N,N',N"-trialkyl melamine and N,N,N',N"-tetraalkyl melamine.

11. Method according to one of the preceding claims, **characterized in that** the formed compound is selected from the group comprising N-mono alkyl urea, N,N'-dialkyl urea and N,N,N'-trialkyl urea.

12. Method according to one of the preceding claims, **characterized in that** the formed compound is selected from the group comprising N-monoalkylthiolurea, N,N'-dialkylthiolurea and N,N,N'-trialkylthiolurea.

13. Method according to one of the preceding claims, **characterized in that** the formed compound is selected from the group comprising N,N'-dialkylbenzoguanamine, N,N,N'-trialkylbenzoguanamine, N,N'-dialkyleacetoguanamine and N,N,N'-trialkylacetoguanamine.

14. Method according to one of the preceding claims, **characterized in that** the formed compound is selected from the group comprising N,N'-di-(hydroxyalkyl)-melamine, N,N',N"-tris-(hydroxyalkyle)-melamine, N,N,N',N"-tetra-(hydroxyalkyl)-melamine, N, N, N', N', N"-penta-(hydroxyalkyl)-melamine and N, N,N',N', N", N"-hexa-(hydroxyalkyl)-melamine.

## Revendications

1. Procédé servant à préparer un composé comprenant au moins un groupe amino au moins monosubstitué, **caractérisé en ce qu'**une substance de départ porteuse au moins d'un groupe amino se présentant sous la forme d'un dérivé de triazine de la formule générale (I) ou sous la forme d'une urée ou d'un dérivé d'urée de la formule générale (II) 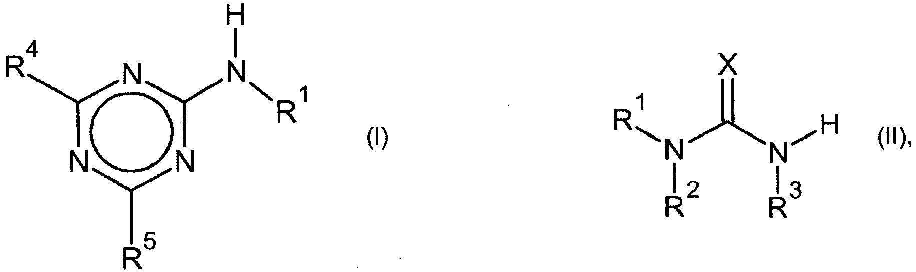 dans laquelle,
- R⁴ et R⁵ signifient, indépendamment l'un de l'autre, Q¹ ou un radical, lié par son atome d'azote central au noyau triazine de la structure de la formule (I), de la formule R⁶-N-R⁷ ou R⁸-N-R⁹, dans laquelle
-- Q¹ représente un alkyle en C₁-C₃₀ linéaire ou ramifié ou un substituant cyclique se présentant sous la forme d'un cycloalkyle en C₅-C₂₀, d'un aryle en C₅-C₂₀, d'un aryle en C₅-C₂₀ substitué par un alkyle en C₁-C₂₀ ou d'un imide d'acides carboxyliques cycliques saturés, dans laquelle l'alkyle en C₁-C₃₀ ou le substituant cyclique peut être interrompu par un ou plusieurs atomes d'oxygène, par un ou plusieurs atomes de soufre, par un ou plusieurs atomes d'azote substitués et/ou par un ou plusieurs groupes du type -C(O)O-, -OC(O), -C(O)- et/ou -OC(O)O,
- R¹, R², R³, R⁶, R⁷, R⁸ et R⁹ signifient, indépendamment les uns des autres, H, un alkyle en C₁-C₂₀ linéaire ou ramifié, un cycloalkyle en C₅-C₂₀, un aryle en C₅-C₂₀, un aryle en C₅-C₂₀ substitué par un alkyle en C₁-C₂₀, qui peut être respectivement interrompu par un ou plusieurs atomes d'oxygène, par un ou plusieurs atomes de soufre, par un ou plusieurs atomes d'azote substitués et/ou par un ou plusieurs groupes du type -C(O)O-, -OC(O)-, -C(O)- et/ou -OC(O)O et/ou fonctionnalisé par un ou plusieurs groupes hydroxy et/ou par un ou plusieurs groupes mercapto, et
- X signifie O ou S,
est mis en réaction, dans un mélange réactionnel en présence d'ammoniaque, avec un alcool de la formule générale R¹⁰-OH, dans laquelle R¹⁰ signifie un alkyle en C₁-C₂₀ linéaire ou ramifié, un cycloalkyle en C₅-C₂₀ ou un aryle en C₅-C₂₀ substitué par un alkyle en C₁-C₂₀, qui peut être respectivement interrompu par un ou plusieurs atomes d'oxygène, par un ou plusieurs atomes de soufre, par un ou plusieurs atomes d'azote substitués et/ou par un ou plusieurs groupes du type -C(O)O-, -OC(O)-, -C(O)- et/ou -OC(O)O- et/ou fonctionnalisé par un ou plusieurs groupes hydroxy et/ou par un ou plusieurs groupes mercapto,
dans lequel le rapport molaire entre l'ammoniaque et l'alcool présente une valeur de 0,1 à 2, en particulier de 0,5 à 1,5 et tout particulièrement de 0,8 à 1,3.

2. Procédé selon la revendication 1, **caractérisé en ce que** la mise en réaction est effectuée à une pression globale allant de 1 à 200 bar, en particulier allant de 40 à 180 bar et tout particulièrement allant de 60 à 140 bar.

3. Procédé selon la revendication 1 ou 2, **caractérisé en ce que** le mélange réactionnel présente un catalyseur, en particulier un catalyseur en métal et/ou en oxyde de métal.

4. Procédé selon l'une quelconque des revendications précédentes, **caractérisé en ce que** la mise en réaction est effectuée à une température allant de 100 °C à 300 °C, en particulier allant de 150 °C à 250 °C, en particulier allant de 180 °C à 240 °C.

5. Procédé selon l'une quelconque des revendications précédentes, **caractérisé en ce que** la mise en réaction est effectuée lors d'une réaction présentant une durée allant de 1 minute à 20 heures, en particulier allant de 1 heure à 10 heures, et tout particulièrement allant de 4 heures à 8 heures.

6. Procédé selon l'une quelconque des revendications précédentes, **caractérisé en ce qu'**au moins un groupe hydroxyle de l'alcool est présent au niveau du radical alkyle et pas au niveau du radical aryle lorsque R¹⁰ représente un aryle en C₅-C₂₀ substitué par un alkyle en C₁-C₂₀.

7. Procédé selon l'une quelconque des revendications précédentes, **caractérisé en ce que** l'alcool est un monoalcool ou un polyalcool.

8. Procédé selon l'une quelconque des revendications précédentes, **caractérisé en ce que** le composé obtenu est un dérivé de triazine de la formule générale (III) ou un dérivé d'urée de la formule générale (IV) : 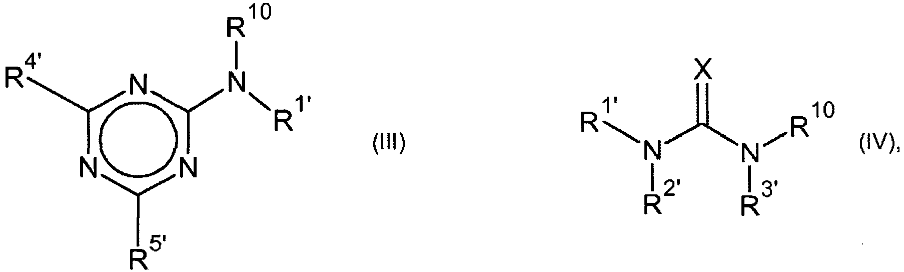 dans laquelle
- R^{4'} et R^{5'} signifient, indépendamment l'un de l'autre, Q¹ ou un radical, lié par son atome d'azote central au noyau de trizaine de la structure de la formule (III), de formule R^{6'}-N-R^{7'} ou R^{8'}-N-R^{9'}, où dans laquelle
- - Q¹ signifie un alkyle en C₁-C₃₀ linéaire ou ramifié ou un substituant cyclique se présentant sous la forme d'un cycloalkyle en C₅-C₂₀, d'un aryle en C₅-C₂₀, d'un aryle en C₅-C₂₀ substitué par un alkyle en C₁-C₂₀ ou d'un imide d'acides carboxyliques cycliques saturés, dans laquelle l'alkyle en C₁-C₃₀ ou le substituant cyclique peut être interrompu par un ou plusieurs atomes d'oxygène, par un ou plusieurs atomes de soufre, par un ou plusieurs atomes d'azote substitués et/ou par un ou plusieurs groupes du type -C(O)O-, -OC(O)-, -C(O)- et/ou -OC(O)O-,
- R^{1'}, R^{2'}, R^{3'}, R^{6'}, R^{7'}, R^{8'} et R^{9'} signifient, indépendamment les uns des autres,
- - R¹⁰,
- - H,
- - une liaison covalente liée au radical R¹⁰ lié au même atome d'azote du dérivé de triazine ou du dérivé d'urée de manière à former une structure cyclique à partir de l'atome d'azote et du radical R¹⁰,
ou
- - un alkyle en C₁-C₂₀ linéaire ou ramifié, un cycloalkyle en C₅-C₂₀, un aryle en C₅-C₂₀, un aryle en C₅-C₂₀ substitué par un alkyle en C₁-C₂₀, qui peut être respectivement interrompu par un ou plusieurs atomes d'oxygène, par un ou plusieurs atomes de soufre, par un ou plusieurs atomes d'azote substitués et/ou par un ou plusieurs groupes du type -C(O)O-, -OC(O)-, -C(O)- et/ou -OC(O)O- et/ou fonctionnalisé par un ou plusieurs groupes hydroxy et/ou par un ou plusieurs groupes mercapto,
- R¹⁰ signifie un alkyle en C₁-C₂₀ linéaire ou ramifié, un cycloalkyle en C₅-C₂₀ ou un aryle en C₅-C₂₀ substitué par un alkyle en C₁-C₂₀, qui peut être respectivement interrompu par un ou plusieurs atomes d'oxygène, par un ou plusieurs atomes de soufre, par un ou plusieurs atomes d'azote substitués et/ou par un ou plusieurs groupes du type -C(O)O-, -OC(O)-, -C(O)- et/ou -OC(O)O- et/ou fonctionnalisé par un ou plusieurs groupes hydroxy et/ou par un ou plusieurs groupes mercapto, dans laquelle une structure cyclique comprenant deux liaisons covalentes liées au même atome d'azote des composés de la formule générale (III) ou (IV) peut être réalisée par **R¹⁰**, une des deux liaisons covalentes étant fournie par les radicaux R^{1'}, R^{2'}, R^{3'}, R^{6'}, R^{7'}, R^{8'} ou R^{9'}, et
- X signifie O ou S.

9. Procédé selon la revendication 8, **caractérisé en ce que** la signification du radical R¹⁰ revient au moins à un radical, en particulier à au moins deux radicaux, en particulier à au moins trois radicaux, en particulier à au moins quatre radicaux, en particulier à au moins cinq radicaux des radicaux R^{1'}, R^{2'}, R^{3'}, R^{6'}, R^{7'}, R^{8'} et R^{9'}.

10. Procédé selon l'une quelconque des revendications précédentes, **caractérisé en ce que** le composé obtenu est choisi parmi le groupe comprenant N,N'-dialkyl-mélamine, N,N',N"-trialkyl-mélamine et N, N, N', N"-tétraalkyl-mélamine.

11. Procédé selon l'une quelconque des revendications précédentes, **caractérisé en ce que** le composé obtenu est choisi parmi le groupe comprenant N-monoalkyl-urée, N,N'-dialkyl-urée et N, N,N'-trialkyl-urée.

12. Procédé selon l'une quelconque des revendications précédentes, **caractérisé en ce que** le composé obtenu est choisi parmi le groupe comprenant N-monoalkylthio-urée, N,N'-dialkylthio-urée et N,N,N'-trialkylthio-urée.

13. Procédé selon l'une quelconque des revendications précédentes, **caractérisé en ce que** le composé obtenu est choisi parmi le groupe comprenant N,N'-dialkylbenzo-guanamine, N,N,N'-trialkylbenzo-guanamine, N,N'-dialkyl-acétoguanamine et N,N,N'-trialkyl-acétoguanamine.

14. Procédé selon l'une quelconque des revendications précédentes, **caractérisé en ce que** le composé obtenu est choisi parmi le groupe comprenant N,N'-di(hydroxyalkyl)-mélamine, N,N',N"-tris-(hydroxyalkyl)-mélamine, N,N,N',N"-tétra-hydroxyalkyl)-mélamine, N,N,N',N',N"-penta-(hydroxyalkyl)-mélamine et N,N,N',N',N",N"-hexa-(hydroxyalkyl)-mélamine.
